# EUROPÄISCHE PATENTSCHRIFT

(11) **EP 0 360 988 B1**
(45) Veröffentlichungstag und Bekanntmachung des Hinweises auf die Patenterteilung: **26.04.1995**
(21) Anmeldenummer: 89112302.8
(22) Anmeldetag: 06.07.1989
(51) Int. Cl.: C07C 47/052

(54) **Verfahren zur Herstellung von wässrigen Formaldehydlösungen**
Process for the manufacture of an aqueous formaldehyde solution
Procédé pour la fabrication d'une solution aqueuse de formaldéhyde

(30) Priorität: 19.07.1988 DE 3824360; 08.10.1988 DE 3834323
(43) Veröffentlichungstag der Anmeldung: 04.04.1990
(73) Patentinhaber: BAYER AG, 51368 Leverkusen (DE)
(72) Erfinder: Alpers, Heinz-Jürgen, D-4150 Krefeld 11 (DE); Dietz, Karl-Heinz, D-4150 Krefeld 1 (DE); Schenke, Bernd-Ulrich, D-4250 Bottrop (DE); Thiel, Reinhard, Dr., D-4150 Krefeld 1 (DE)

(56) Entgegenhaltungen:
- EP-A- 0 100 809
- EP-A- 0 150 436
- DE-A- 1 618 413
- US-A- 4 594 457
- PATENT ABSTRACTS OF JAPAN vol. 2, no. 59 (C-78)(374) 27. April 1978

## Beschreibung

Die vorliegende Erfindung betrifft ein Verfahren zur Herstellung von wäßrigen Formaldehydlösungen, denen der größte Teil der bei ihrer Herstellung anfallenden Kondensations- und Absorptionsenergie zur Verdampfung des benötigten Methanol/Wasser/Luft-Gemisches entzogen wird. Weiterhin kann ein Teil des Wärmeinhaltes der hergestellten Formaldehydlösung zur Wiedergewinnung von in der Formaldehydlösung enthaltenem Methanol genutzt werden. Beide Maßnahmen können sowohl allein als auch gleichzeitig angewandt werden.

Formaldehyd ist eine wichtige Grundchemikalie, beispielsweise zur Herstellung von Phenol-Formaldehyd-Harzen oder Harnstoff-Formaldehyd-Harzen und wird weiterhin als Desinfektionsmittel und als Gerbstoff eingesetzt.

Die Herstellung von Formaldehyd durch oxidierende Dehydrierung von Methanol ist seit langem bekannt. Hierbei werden vollständig verdampfte Gemische aus Methanol, Wasser und Luft, deren Zusammensetzung in weiten Grenzen außerhalb des Bereichs explosiver Gemische variieren kann, an Katalysatoren bei erhöhter Temperatur umgesetzt. Als Katalysatoren kommen solche aus Silber und anders in Frage. Das der Katalysatorstufe entströmende gasförmige Gemisch wird sodann in Absorbern in wäßrige Formaldehydlösungen umgewandelt. Hierbei muß noch ein beträchtlicher Teil des Wärmeinhalts der zu absorbierenden Gase und die Absorptions-und Kondensationswärme abgeführt werden. Es besteht nun der Wunsch, diese auf niedrigen Temperaturniveau anfallende Energie wirtschaftlich zu nutzen. Das zur Herstellung von Formaldehydlösungen benötigte Methanol/Wasser/ Luft-Gemisch wird gemeinhin in einem Verdampfer durch Beheizung mit Dampf hergestellt.

Es wurde nun überraschend gefunden, daß man die vollständige Verdampfung des Methanol/Wasser/Luft-Gemisches in einem geeigneten Verdampfer unterhalb des Siedepunktes der Methanol/Wasser-Mischung durchführen kann und daß zur Beheizung die Temperatur der im Absorber anfallenden wäßrigen Formaldehydlösung ausreicht.

Daneben besteht der Wunsch, aus der hergestellten Formaldehydlösung sowohl das Methanol weitgehend zu entfernen und wieder zu verwenden, als auch einen Teil des Wassers zu entfernen, um höherkonzentrierte Formaldehydlösungen, beispielsweise zur Einsparung von Transportkosten, zur Verfügung zu haben. Diese Entfernung von Methanol und Wasser wird destillativ, bevorzugt unter vermindertem Druck, ausgeführt. Eine solche Destillation unter vermindertem Druck wird vorteilhaft als Entspannungsverdampfung unter Ausnutzung der in den rohen Formaldehydlösungen enthaltenen Wärme durchgeführt (EP 100 809). Je weiter der Druck bei einer solchen Entspannungsverdampfung abgesenkt wird, desto weiter sinkt die Kolonnentemperatur, was einer immer weitergehenden und wünschenswerten Energieausnutzung entspricht. Diese Energieausnutzung hat jedoch vom wirtschaftlichen Standpunkt ihre Grenzen, da bei tieferen Temperaturen die Brüdenkondensation nur durch stark vergrößerte Kondensatorflächen und durch die Benutzung aufwendiger Kühlmittel, wie einer Kühlsole, gelingt. Hierdurch wird der wirtschaftliche Vorteil der Energieausnutzung aufgezehrt und schließlich ins Gegenteil verkehrt.

Es wurde nun weiterhin gefunden, daß man auch bei sehr tiefen Drücken in der Entspannungskolonne auf den Einsatz kostenaufwendiger Kühlmittel verzichten kann, wenn die Kondensation der Brüden als Einspritzkondensation mit flüssigem Wasser vorgenommen wird und die dabei anfallende Methanol-haltige wässrige Lösung mit einer geringen Konzentration an Formaldehyd, die entsorgungsbedürftig ist, anstelle des als Einsatzprodukt benötigten Wassers der katalytischen Dehydrierung des Methanols zugeführt wird.

Durch die Möglichkeit, bei sehr niedrigen Kolonnendrücken zu arbeiten, gelingt es auch, beide Maßnahmen gleichzeitig durchzuführen und so eine optimale Nutzung der in der Formaldehydlösung enthaltenen Wärme zu erzielen.

Vorteile einer solchen Verfahrensweise sind:
1. Die Einsparung des zur Verdampfung des Methanol/Wasser/Luft-Gemisches benötigten Dampfes;
2. eine Einsparung von Methanol unter weitgehender Energieausnutzung, insbesondere im unteren Druckbereich der Entspannungsverdampfung, so daß das Verfahren ohne Fremdenergie auskommt;
3. die Möglichkeit, auch niedriger konzentrierte Methanol/Wasser-Mischungen einzusetzen und
4. es besteht die Möglichkeit, auf einen zu hohen Methanolumsatz zu verzichten.

Die unter 3. und 4. genannten Maßnahmen fördern die Selektivität der katalytischen Dehydrierung und damit die Ausbeute und sind nur möglich, weil eine weitgehende Methanolrückgewinnung auf wirtschaftliche Weise erfindungsgemäß möglich ist.

Eine bisher aus JP-A 53/15 307 (1978) bekannt gewordene Möglichkeit zur Nutzung der Abwärme beschränkt sich auf die Verdampfung von Methanol allein, offenbar weil der hohe Taupunkt eines Methanol/Wasser-Gemisches eine gemeinsame Verdampfung durch die Abwärme des Absorbers nicht durchführbar erscheinen ließ.

DE-OS 1 618 413 beschreibt eine aufwendige Nutzung der in den Kondensationsbrüden enthaltenen Wärme durch einen als Wärmepumpe dienenden Brüdenverdichter, der mit elektrischer Energie betrieben wird und somit einen Teil der zurückgewonnenen Energie wieder verbraucht.

In EP-A 150 436 wird die Absorptions- und Reaktionswärme zum Erwärmen einer Kreislaufflüssigkeit, die Wasser und Methanol enthält, benutzt. Aus der Kreislaufflüssigkeit wird durch Strippen mit Luft, Inertgas und/oder Abgas ein Methanol/Wasser-Gemisch abgetrennt.

Die Erfindung betrifft demnach ein Verfahren zur Herstellung von wäßrigen Formaldehydlösungen, bestehend aus den Stufen
a) Umsetzung eines dampfförmigen Methanol/Wasser/Luft-Gemisches bei höherer Temperatur an einem Katalysator,
b) Absorption des Umsetzungsgemisches in einer oder mehreren hintereinandergeschalteten Absorptionsstufen unter Bildung einer methanolhaltigen wäßrigen Formaldehydlösung und
c) fraktioniertes Abdestillieren einer im wesentlichen Methanol und Wasser enthaltenden Fraktion aus dieser Formaldehydlösung,
das dadurch gekennzeichnet ist, daß das zur Umsetzung benötigte Methanol/Wasser-Gemisch durch indirekte Beheizung mit einer dem Absorber entnommenen Formaldehydlösung verdampft wird und daß mindestens ein Teil der beim fraktionierten Abdestillieren erhaltenen Brüden mit flüssigem Wasser kondensiert wird und dieses Kondensat mit frischem Methanol und mit Luft dem der Katalysatorstufe vorgeschalteten Verdampfer zugeführt wird.

In einer bevorzugten Variante wird im erfindungsgemäßen Verfahren die Absorption in mindestens zwei hintereinander geschalteten Absorptionsstufen vorgenommen. Die für das fraktionierte Abdestillieren eingesetzte methanolhaltige wäßrige Formaldehydlösung wird in diesem Fall der zweiten Absorptionsstufe oder einer der weiteren nachgeschalteten Absorptionsstufen entnommen; die zur Beheizung des der Katalysatorstufe vorgeschalteten Verdampfers erforderliche Formaldehydlösung wird der ersten Absorptionsstufe oder einer der vorgeschalteten Absorptionsstufen, bevorzugt der ersten Absorptionsstufe, entnommen. Die vom Verdampfer zurücklaufende Formaldehydlösung kann sowohl als Verfahrensprodukt zur weiteren Verwendung entnommen werden, als auch der Absorptionsstufe, der sie entnommen wurde, wieder zugeführt werden. Die indirekte Beheizung durch dem Absorber entnommene Formaldehydlösung deckt den gesamten oder mindestens überwiegenden Energiebedarf des Verdampfers. Daneben ist eine Zusatzbeheizung zum Anfahren des Verfahrens installiert.

Ungeachtet des hier benutzten Sprachgebrauchs von einem Absorber pro Absorptionsstufe kann selbstverständlich auch jede Absorptionsstufe auf mehreren Absorbern bestehen.

Ein geeigneter Verdampfer ist so konstruiert, daß eine intensive und über seinen Querschnitt gleichmäßige Verteilung der Komponenten Methanol/Wasser und Luft gewährleistet ist. Ein so konstruierter Verdampfer kann ohne Fremdenergie, d.h. nur mit Hilfe der dem Absorber entnommenen Formaldehydlösung, beheizt werden; die in der Absorptionsstufe anfallende Absorptions- und Kondensationsenergie kann weitgehend genützt werden.

Das fraktionierte Abdestillieren von im wesentlichen Methanol und Wasser (neben geringen Mengen an Formaldehyd) kann bei einem Druck von 40 bis 800 mbar, gemessen am Kolonnenkopf, durchgeführt werden. Bevorzugt wird dabei ein Druck von 50 bis 400 mbar, besonders bevorzugt von 50 bis 200 mbar und ganz besonders bevorzugt von 50 bis 95 mbar, alles gemessen am Kolonnenkopf, gearbeitet.

Die bei der fraktionierten Destillation erhaltenen Brüden werden nur zu einem Teil an einem Oberflächenkondensator kondensiert, um einen Rückfluß in gewünschtem Umfange für die Destillationskolonne aufrecht zu erhalten. Der größte Teil des Methanols verläßt diesen Oberflächenkondensator noch in dampfförmigen Zustand und wird in einem nachgeschalteten Einspritzkondensator durch zugefügtes Wasser vollständig niedergeschlagen. Das hierbei erhaltene Kondensat wird dem der katalysatorstufe vorgeschalteten Verdampfer zugeführt. Für die Einspritzkondensation kann somit bevorzugt eine Wassermenge eingesetzt werden, die bis zur gesamten im Methanol/Wasser/Luft-Gemisch benötigte Wassermenge reicht.

Obwohl in der beschriebenen Weise die in den Absorptionslösungen enthaltene fühlbare Wärme anderen Wärmeverbrauchern des erfindungsgemäßen Verfahrens (Verdampfer und/oder fraktionierte Destillation) zugeführt wird, so daß diese ohne Fremdenergie betrieben werden können, ist es dennoch möglich, die der Katalysatorstufe entströmenden Gase einem an sich bekannten Abhitzekessel zuzuführen, ohne daß Energiemangel an den anderen beschriebenen Stellen auftritt. Es ist daher eine weitere bevorzugte Variante, die Dampferzeugung in einem solchen Abhitzekessel auch im erfindungsgemäßen Verfahren durchzuführen.

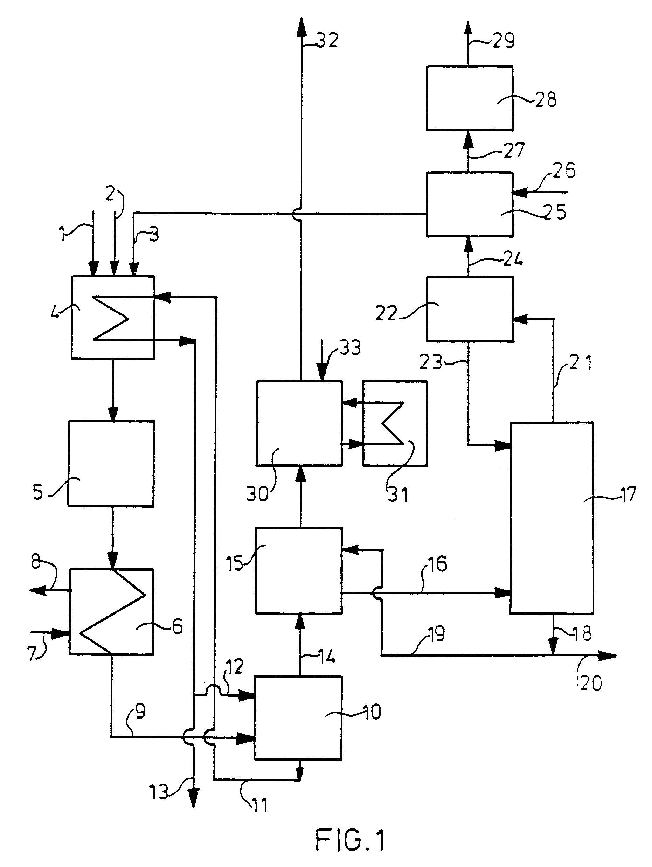

Anhand der beigefügten Fig. 1 sei das erfindungsgemäße Verfahren am Beispiel einer dreistufigen Absorption wie folgt erläutert, wobei der Verdampfer durch aus dem Absorber entnommene Formaldehydlösung beheizt wird, die Brüden der Entspannungsverdampfung durch Frischwasser niedergeschlagen werden und zusätzlich ein Abhitzekessel betrieben wird:
Frisches Methanol (1), Luft (2) und ein recyclisiertes, im wesentlichen aus Methanol und Wasser bestehendes Gemisch (3) wird im Verdampfer (4) vollständig in die Dampfform übergeführt und der Katalysatorstufe (5) zugeführt. Das der Katalysatorstufe (5) entströmende Reaktionsgemisch wird einem Abhitzekessel (6) zugeleitet, wo es auf ca. 120°C abgekühlt wird unter gleichzeitiger Umwandlung von gegebenenfalls vorgewärmtem Wasser (7) in einen 5- bis 6-bar-Dampf (8). Das den Abhitzekessel verlassende Reaktionsgemisch wird über die Leitung (9) einer ersten Absorberstufe (10) zugeleitet. Die in (10) entstehende wäßrige Formalinlösung wird über die Leitung (11) dem Verdampfer (4) zum Wärmeaustausch zugeführt und teilweise über die Leitung (12) wieder in die erste Absorberstufe (10) zurückgeleitet. Ein Teil der wäßrigen Formalinlösung aus (10) wird nach dem Wärmeaustausch (4) als Produktstrom (13) entnommen. Die in (10) nicht kondensierten Reaktionsgase werden über die Leitung (14) der zweiten Absorberstufe (15) zugeleitet. Die (15) entströmende methanolhaltige wäßrige Formaldehydlösung wird über die Leitung (16) in die Rektifizierkolonne (17) eingespeist, wo unter vermindertem Druck eine Entspannungsverdampfung stattfindet. Die zurückbleibende methanolarme wäßrige Formaldehydlösung wird am Sumpf von (17) über die Leitung (18) entnommen und zum Teil als Rücklauf zur zweiten Absorberstufe (15) über die Leitung (19) geleitet und zum Teil über die Leitung (20) als Produkt abgenommen. Die in (17) entstehenden Brüden, die hauptsächlich aus Methanol und Wasser neben geringen Mengen Formaldehyd bestehen, werden über die Leitung (21) einem Oberflächenkondensator (22) zugeführt, wo sie lediglich zum Teil kondensiert werden, um über die Leitung (23) als Rückfluß auf die Kolonne (17) geleitet zu werden. Ein Teil der Brüden bleibt im dampfförmigem Zustand und wird über die Leitung (24) einem Einspritzkondensator (25) zugeführt, worin alle kondensierbaren Anteile der Brüden mit Hilfe von durch die Leitung (26) eingespeistem Wasser niedergeschlagen werden. Das Kondensat wird über die Leitung (3) dem Verdampfer (4) zugeleitet. Die nicht kondensierbaren Anteile und die Inertgasbestandteile werden über die Leitung (27) der Vakuumpumpe (28) zugeleitet, an deren Druckseite sie als Abgas (29) entweichen und einer geeigneten Entsorgung zugeführt werden. Der in der zweiten Absorberstufe (15) nicht absorbierte Teil der Reaktionsgase wird in einer dritten Absorberstufe (30) absorbiert. Die in diesem Absorbat noch aufgenommene Kondensationswärme wird durch einen Kühler (31) abgeführt. Notwendiges Wasser als Absorptionsmedium wird über (33) bereitgestellt. Die nicht kondensierbaren Bestandteile und die Inertgase werden als Abgas (32) einer geeigneten Entsorgung, in der Regel einer Verbrennung unter Dampferzeugung, zugeführt.

### Beispiel 1

In einer Anlage gemäß Fig. 1 werden stündlich 583 Gew.-Teile Methanol, 970 Gew.-Teile Luft und 480 Gew.-Teile Kondensat, bestehend aus 17 Gew.-Teilen Methanol, 460 Gew.-Teilen Wasser und Restmengen Formaldehyd, im Verdampfer (4) vollständig verdampft und dem Reaktor (5), der mit einem Silberkatalysator ausgestattet ist, zugeleitet. Im Abhitzekessel (6) werden stündlich ca. 630 Gew.-Teile Dampf von 5 bar produziert und über Leitung (8) dem allgemeinen Dampfnetz zugeführt. Die Reaktionsgase kühlen sich dabei ca. 120°C ab. Aus der ersten Absorptionsstufe zirkulieren ca. 25.000 Gew.-Teile/h wäßrige Formalinlösung von 79-83°C über Leitung (11) zum Verdampfer (4), die beim Durchgang durch den Verdampfer (4) auf 68-70°C heruntergekühlt werden. Hiervon werden stündlich über die Leitung (13) 570 Gew.-Teile wäßrige Formaldehydlösung abgenommen (40,5 % Formaldehyd und ca. 1 % Methanol). Aus der zweiten Absorptionsstufe (15) zirkulieren ca. 27.000 Gew.-Teile wäßrige Formaldehydlösung zur Rektifizier-/Entspannungskolonne (17), von denen nach Entspannungsverdampfung bei 85 mbar Kopfdruck über (20) 880 Gew.-Teile Formaldehydlösung/h entnommen werden (30,5 % Formaldehyd und 0,5 % Methanol). Die den Oberflächenkondensator (22) durchströmenden Brüden werden im Einspritzkondensator (25) mit 450 Gew.-Teilen Wasser/h (über (26)) niedergeschlagen und ergeben ein zum Verdampfer (4) zurückfließendes Kondensat mit der oben angegebenen Zusammensetzung. Die Ausbeute an Formaldehyd beträgt, bezogen auf das eingesetzte Methanol, 91,3 % der theoretischen Ausbeute.

## Patentansprüche

1. Verfahren zur Herstellung von wäßrigen Formaldehydlösungen, bestehend aus den Stufen
a) Umsetzung eines dampfförmigen Methanol/Wasser/Luft-Gemisches bei höherer Temperatur an einem Katalysator,
b) Absorption des Umsetzungsgemisches in einer oder mehreren hintereinandergeschalteten Absorptionstufen unter Bildung einer methanolhaltigen wäßrigen Formaldehydlösung und
c) fraktioniertes Abdestillieren einer im wesentlichen Methanol und Wasser enthaltenden Fraktion aus dieser Formaldehydlösung,
dadurch gekennzeichnet, daß das zur Umsetzung benötigte Methanol/Wasser-Gemisch durch indirekte Beheizung mit einer dem Absorber entnommenen Formaldehydlösung verdampft wird und daß mindestens ein Teil der beim fraktionierten Abdestillieren erhaltenen Brüden mit flüssigem Wasser kondensiert wird und dieses Kondensat mit frischem Methanol und mit Luft dem der Katalysatorstufe vorgeschalteten Verdampfer zugeführt wird.

2. Verfahren nach Anspruch 1, dadurch gekennzeichnet, daß die Absorption in mindestens zwei hintereinandergeschalteten Absorptionsstufen vorgenommen wird und die der ersten Absorptionsstufe entnommene Formaldehydlösung dem Verdampfer zum Wärmeaustausch zugeführt wird und daß die für das fraktionierte Abdestillieren eingesetzte methanolhaltige wäßrige Formaldehydlösung einer nachgeschalteten Absorptionsstufe, bevorzugt der zweiten Absorptionsstufe, entnommen wird.

3. Verfahren nach Anspruch 1, dadurch gekennzeichnet, daß das fraktionierte Abdestillieren bei einem Druck von 40 bis 800 mbar, gemessen am Kolonnenkopf, durchgeführt wird.

4. Verfahren nach Anspruch 3, dadurch gekennzeichnet, daß das fraktionierte Abdestillieren bei einem Druck von 50 bis 400 mbar, gemessen am Kolonnenkopf, durchgeführt wird.

5. Verfahren nach Anspruch 4, dadurch gekennzeichnet, daß das fraktionierte Abdestillieren bei einem Druck von 50 bis 200 mbar, gemessen am Kolonnenkopf, durchgeführt wird.

6. Verfahren nach Anspruch 5, dadurch gekennzeichnet, daß das fraktionierte Abdestillieren bei einem Druck von 50 bis 95 mbar, gemessen am Kolonnenkopf, durchgeführt wird.

7. Verfahren nach Anspruch 1, dadurch gekennzeichnet, daß das der Katalysatorstufe entströmende Gemisch vor der Absorption einem Abhitzekessel zugeführt wird.

## Claims

1. Process for the preparation of aqueous formaldehyde solutions, consisting of the steps
a) reaction of a methanol/water/air mixture in vapour form at elevated temperature over a catalyst,
b) absorption of the reaction mixture in one or more successive absorption stages with the formation of a methanol-containing aqueous formaldehyde solution and
c) removal by fractional distillation of a fraction essentially containing methanol and water from this formaldehyde solution,
characterized in that the methanol/water mixture required for the reaction is evaporated by indirect heating by means of a formaldehyde solution removed from the absorber and in that at least some of the vapours obtained by fractional distillation are condensed by means of liquid water and this condensate is fed together with fresh methanol and with air into the evaporator which is upstream from the catalyst stage.

2. Process according to Claim 1, characterized in that the absorption is carried out in at least two successive absorption stages and the formaldehyde solution removed from the first absorption stage is fed into the evaporator for the purpose of heat-exchange and in that the methanol-containing aqueous formaldehyde solution which is used for the fractional distillation is removed from a downstream absorption stage, preferably the second absorption stage.

3. Process according to Claim 1, characterized in that the fractional distillation is carried out at a pressure of 40 to 800 mbar, measured at the column head.

4. Process according to Claim 3, characterized in that the fractional distillation is carried out at a pressure of 50 to 400 mbar, measured at the column head.

5. Process according to Claim 4, characterized in that the fractional distillation is carried out at a pressure of 50 to 200 mbar, measured at the column head.

6. Process according to Claim 5, characterized in that the fractional distillation is carried out at a pressure of 50 to 95 mbar, measured at the column head.

7. Process according to Claim 1, characterized in that the mixture which leaves the catalyst stage is fed into a waste heat boiler before the absorption.

## Revendications

1. Procédé de préparation de solutions aqueuses de formaldéhyde, consistant en les stades opératoires suivants :
a) conversion d'un mélange méthanol/eau/air, à l'état de vapeurs, à température élevée, sur un catalyseur,
b) absorption du mélange de réaction dans un ou plusieurs étages d'absorption successifs avec formation d'une solution aqueuse méthanolique de formaldéhyde et
c) distillation fractionnée d'une fraction de cette solution de formaldéhyde contenant essentiellement du méthanol et de l'eau,
caractérisé en ce que le mélange de départ méthanol/eau est vaporisé par chauffage indirect à l'aide d'une solution de formaldéhyde prélevée à l'absorbeur, une partie au moins des vapeurs obtenues à la distillation fractionnée sont condensées à l'aide d'eau à l'état liquide et ce condensat est recyclé avec du méthanol frais et avec de l'air au vaporiseur précédant l'étage catalyseur.

2. Procédé selon la revendication 1, caractérisé en ce que l'absorption est réalisée dans au moins deux étages d'absorption successifs, en ce que la solution de formaldéhyde prélevée au premier étage d'absorption est envoyée au vaporiseur pour échange de chaleur et en ce que la solution aqueuse méthanolique de formaldéhyde mise en oeuvre à la distillation fractionnée est prélevée dans un étage d'absorption consécutif, de préférence au deuxième étage d'absorption.

3. Procédé selon la revendication 1, caractérisé en ce que la distillation fractionnée est réalisée sous une pression de 40 à 800 mbar, la mesure étant faite en tête de colonne.

4. Procédé selon la revendication 3, caractérisé en ce que la distillation fractionnée est réalisée sous une pression de 50 à 400 mbar, la mesure étant faite en tête de colonne.

5. Procédé selon la revendication 4, caractérisé en ce que la distillation fractionnée est réalisée sous une pression de 50 à 200 mbar, la mesure étant faite en tête de colonne.

6. Procédé selon la revendication 5, caractérisé en ce que la distillation fractionnée est réalisée sous une pression de 50 à 95 mbar, la mesure étant faite en tête de colonne.

7. Procédé selon la revendication 1, caractérisé en ce que le mélange sortant de l'étage catalyseur est envoyé à une chaudière à récupération de chaleur avant l'absorption.
